# EUROPÄISCHE PATENTSCHRIFT

(11) **EP 3 138 584 B1**
(45) Veröffentlichungstag und Bekanntmachung des Hinweises auf die Patenterteilung: **03.07.2019**
(21) Anmeldenummer: 16193504.4
(22) Anmeldetag: 12.04.2011
(51) Int. Cl.: A61L 27/18, A61L 27/30

(54) **IMPLANTAT UND VERFAHREN ZUR HERSTELLUNG EINES IMPLANTATES**
IMPLANT AND METHOD FOR PRODUCING AN IMPLANT
IMPLANT ET PROCÉDÉ DE FABRICATION D'UN IMPLANT

(30) Priorität: 15.04.2010 DE 102010015099
(43) Veröffentlichungstag der Anmeldung: 08.03.2017
(62) Teilanmeldung aus: 11715205.8
(73) Patentinhaber: Advanced Medical Technologies AG, 66620 Nonnweiler-Braunshausen (DE)
(72) Erfinder: STUDER, Armin, 6330 Cham (CH); FORGAS, Jorge Garcia, 8424 Embrach (CH); SALITO, Armando, 5610 Wohlen (CH)
(74) Vertreter: Viering, Jentschura & Partner mbB Patent- und Rechtsanwälte

(56) Entgegenhaltungen:
- EP-A1- 1 647 242
- EP-A1- 2 199 423

## Beschreibung

Die Erfindung betrifft ein Implantat mit einem Grundkörper und einer auf mindestens einem Oberflächenabschnitt des Grundkörpers aufgebrachten Doppelbeschichtung gemäß Patentanspruch 1.

Bekanntermaßen werden viele Implantate, Prothesen oder Endoprothesen aus Polyetheretherketon (PEEK) gefertigt. Dieses Material besitzt gegenüber metallischen Materialien den Vorteil, dass das E-Modul von PEEK vielmehr dem E-Modul von kortikalen Knochen entspricht, als metallische Materialien dies je erreichen können. Außerdem ist PEEK röntgenstrahlendurchlässig, sodass der Mediziner bei Nachkontrollen durch entsprechende Röntgenaufnahmen ein knöchernes Durchwachsen von beispielsweise Wirbelcages beobachten kann. Bei einem Titan-Cage wäre dies nicht möglich.

Seit einiger Zeit sind jedoch auch Implantate und dergleichen, welche aus PEEK gefertigt sind, in Kritik geraten. Es konnte beobachten werden, dass der menschliche oder tierische Knochen nicht vollständig an das Implantat anwächst bzw. in dieses hineinwächst. Die Knochen bilden vielmehr an der Oberfläche des PEEK-Materials einen Saum. Kann eine derartige Saumbildung auf einem Röntgenbild festgestellt werden, bedeutet dies, dass ein knöchernes Anwachsen nicht erfolgt ist und keine ausreichende Stabilität bzgl. des eingesetzten Implantates gegeben ist.

In Folge dessen ist entweder das Implantat zu entfernen und durch ein neues zu ersetzen oder durch andere chirurgische Verfahren fest am Knochen zu fixieren. Eine erneute Operation ist mit zusätzlichem Stress, Schmerzen und den entsprechenden Operationsrisiken für den Patienten verbunden.

Metallisches Material, insbesondere Titan, erfüllt optimale Bedingungen bzgl. des Einwachsens in tierische oder menschliche Knochenstrukturen. Nachweislich wachsen die Knochen an das Titan an und sofern die Oberfläche entsprechend gestaltet ist, kann der Knochen auch in die Mikrostrukturen von Titanmaterialien einwachsen.

Daher werden seit längerem Versuche unternommen, Beschichtungen bzw. Implantatmaterialen derartig weiter zu entwickeln, sodass zum einen eine verbesserte bioaktive Oberflächenschicht und ein damit verbundenes Einwachsvermögen für tierische oder menschliche Knochen erreicht wird und zum anderen, wie bereits durch die Verwendung von PEEK-Materialien realisierte, gute E-Module zu erlangen sind.

In der EP 1 372 749 B1 wird eine bioaktive Oberflächenschicht für Implantate und Prothesen offenbart, wobei das Implantat aus PEEK bestehen kann. Ein variabler Anteil der Oberflächenschicht besteht aus Calciumphosphatphasen, wobei die in der Oberflächenschicht eingelagerten Ca-Ionen und P0₄-Ionen vollständig über eine Metalloxid-Schicht verteilt sind. Bei dem Metalloxid handelt es sich beispielsweise um Titandioxid. Des Weiteren wird ein zusätzliches Beschichten der Oberflächenschicht mit Hydroxylapatit beschrieben. Derartige Hydroxylapatitbeschichtungen von Implantaten sind gängige Methoden um ein verbessertes Einwachsen von Knochenstrukturen in das Implantat zu gewährleisten.

EP 1 647 242 offenbart ein Implantat, welches ein Grunkörper und eine auf einem Oberflächenabschnitt des Grundkörpers aufgebrachte Beschichtung aufweist. Der Grundkörper besteht aus Polyetheretherketon und die Beschichtung besteht aus Reintitan, welches mittels eines vakuumbasierten Beschichtungsverfahren aufgebracht ist. Die Titan-Schicht ist so deponiert, dass sie von der inneren Seite nach der äußeren Seite ein Gradient in der Porosität aufweist.

Testungen der zu erreichenden Zugfestigkeitswerte und der zu widerstehenden Scherkrafte von gängigen Implantaten lassen jedoch den Wunsch nach hinsichtlich der beiden Werte verbesserten Implantaten wachsen, wobei die Implantate jedoch auch derartig gut in tierische oder menschliche Knochenstrukturen einwachsen sollen, wie dies beispielweise bei einer Implantatbeschichtung mit Hydroxylapatit der Fall ist.

Aus dem Vorgenannten ist es daher die Aufgabe der vorliegenden Erfindung ein verbessertes Implantat zur Verfügung zu stellen, welches eine kostengünstig zu realisierende Beschichtung aufweist, verbesserte Zugfestigkeitswerte besitzt sowie mit höheren Scherkräften belastet werden kann. Des Weiteren soll mit der vorliegenden Erfindung ein Verfahren angegeben werden, mit Hilfe dessen eine schnelle und kostengünstige Produktion eines beschichteten Implantats verwirklicht werden kann.

Die Lösung der Aufgabe erfolgt durch ein Implantat mit einem Grundkörper und einer auf mindestens einem Oberflächenabschnitt des Grundkörpers aufgebrachten Doppelbeschichtung, gemäß der Merkmalskombination des Patentanspruchs 1, wobei die Unteransprüche mindestens zweckmäßige Ausgestaltungen und Weiterbildungen darstellen.

Erfindungsgemäß weist ein Implantat einen Grundkörper auf, wobei auf der Oberfläche des Grundkörpers partiell eine Doppelbeschichtung aufgebracht ist. Es ist zwar denkbar, die Doppelbeschichtung auf der gesamten Oberfläche des Implantatgrundkörpers aufzubringen, aus Kosten- und Dimensionierungsgründen bzgl. der Gesamtdicke eines Implantats sollten jedoch lediglich einzelne Oberflächenabschnitte oder nur ein Oberflächenabschnitt des Grundkörpers mit der erwähnten Beschichtung versehen werden. Die zu beschichtenden Oberflächenabschnitte sind vor dem Herstellungsverfahren zu berechnen und zwar abhängig von dem herzustellenden Implantat und der Größe des Implantats.

Bei dem Implantat kann es sich beispielsweise um Wirbelcages, Knie- und Hüftprothesen bzw. -endoprothesen, Knochenprothesen oder künstliche Schultergelenke handeln. Das erfindungsgemäße Implantat spielt vor allen Dingen in der zementfreien Prothetik aber auch in der Zahnprothetik eine Rolle.

Bei der Festlegung der Oberflächenabschnitte spielt auch die Größe des zu operierenden Patienten eine Rolle. So besitzen Implantate, Prothesen und Endoprothesen abhängig von Geschlecht, Größe oder Gewicht des Patienten unterschiedliche Abmaße. Auch bei Implantaten zum Einsetzen in tierische Körper werden oftmals unterschiedliche Implantatgrößen zur Verfügung gestellt. Abhängig von der späteren Belastung, Spannung und auf das Implantat im eingesetzten Zustand einwirkenden Scherkräften sind der oder die zu beschichtende(n) Oberflächenabschnitt(e) zu dimensionieren. So kann es in einigen Fällen ausreichend sein, einen einzigen, durchgehenden Oberflächenabschnitt zu bestimmen, allerdings ist es auch denkbar mehrere voneinander beabstandete Oberflächenabschnitte zu definieren.

Der mindestens eine zu beschichtende Oberflächenabschnitt des Implantatgrundkörpers weist im eingesetzten Zustand, d. h. wenn das Implantat in den menschlichen oder tierischen Körper eingebracht ist, zu einem Knochen des
Körpers. Der oder die Oberflächenabschnitt(e) bilden mit der aufzubringenden Doppelbeschichtung die an dem Knochen anliegende Fläche(n) des Implantats.

Die erwähnte Doppelbeschichtung besteht zunächst aus einer Zwischenschicht bzw. Haftvermittlerschicht, welche direkt auf die festgelegten Oberflächenabschnitte bzw. den festgelegten Oberflächenabschnitt des Implantatgrundkörpers aufgebracht ist.

Diese Haftvermittlerschicht ist vollflächig mit einer osteointegrativen Schicht bedeckt. Beide Schichten, d. h. sowohl die Haftvermittlerschicht als auch die osteointegrative Schicht bestehen aus Reintitan.

Bei der Haftvermittlerschicht wird eine Schichtdicke von 2 - 6 µm, insbesondere eine Dicke von 3 - 5 µm, angestrebt. Die osteointegrative Schicht weist eine Schichtdicke von 50 - 70 µm, insbesondere von 55 - 65 µm, auf. Sofern mehrere zu beschichtende Oberflächenabschnitte des Implantatgrundkörpers bestimmt sind, sind die voneinander getrennten Abschnitte mit jeweils der gleichen Schichtdicke bzgl. der Haftvermittlerschicht und der osteointegrativen Schicht zu versehen.

Erfindungsgemäß besitzt die osteointegrative Schicht eine Porosität von 70 - 90 % und eine Rauheit R_{z} von mindestens 45 µm. D. h., der Rauheitswert R_{z} beträgt mindestens 45 µm, kann jedoch größer, z. B. 55 µm sein.

Der Grundkörper des Implantats ist vorzugsweise aus Polyetheretherketon (PEEK) gefertigt, wobei der Grundkörper auch aus anderen Kunststoffen wie beispielsweise Polyoxymethylen (POM), Polyaryletherketon (PAEK), Polyetherimid (PEI), Polymethylpenten (PMP), Polyäthersulfon (PES), Polysulfon (PSU), Polymethylmethacrylat (PMMA) oder Polyäthylenterephthalat (PETP) bestehen kann.

Da wie beschrieben, die osteointegrative Schicht aus reinem Titan gefertigt ist, und erfahrungsgemäß Implantatbeschichtungen aus andersartigem Material beispielsweise zum Abplatzen oder Abbrechen neigen, ist es erfindungsgemäß die Aufgabe der Haftvermittlerschicht eine stabile Verbindung zwischen der osteointegrativen Schicht und dem Implantatgrundkörper herzustellen. Vor allem durch die gewählte Porosität von 70 - 90 % bzgl. der osteointegrativen Schicht, würde eine derartige Schicht, direkt aufgetragen auf die vorher definierten Oberflächenabschnitte des PEEK-Implantatgrundkörpers keine ausreichende bzw. keine dauerhafte Anhaftung erlangen.

Da auch die Haftvermittlerschicht aus reinem Titan besteht, erfolgt das Anhaften der osteointegrativen Schicht auf der Haftvermittlerschicht problemlos. Die Haftvermittlerschicht weist eine große Dichte auf, auf welche die abschließende poröse Schicht - die osteointegrative Schicht - aufgetragen ist.

Die poröse Schicht, auch Porous Coating genannt, sorgt für eine exzellente sofortige Implantatstabilitat sowie für ein hervorragendes Anwachsen des tierischen oder menschlichen Knochens an das Implantat. Auch nach einer bereits längeren Implantationszeit von mehreren Jahren kann keine Ermüdungserscheinung bzgl. der Implantatstabilitat festgestellt werden.

In einer besonders bevorzugten Ausführungsform des erfindungsgemäßen Implantats weist die Haftvermittlerschicht eine Dicke von im Wesentlichen 3 µm auf, wobei im Wesentlichen in diesem Fall bedeutet, dass Abweichung von ± 0,5 µm möglich sind.

Die osteointegrative Schicht besitzt vorzugsweise eine Schichtdicke von im Wesentlichen 60 µm. Bei dieser Schichtdicke liegen Abweichungen von ± 3 µm im Bereich des Möglichen.

Mit den genannten Schichtdicken können besonders gute Resultate betreffend die Zugfestigkeitswerte sowie der möglicherweise einwirkenden Scherkrafte erzielt werden.

Es sei des Weiteren erwähnt, dass eine osteointegrative Schicht mit einer Porosität von 80 % ± 5 % besonders bevorzugt wird.

Das erfindungsgemäße Verfahren zur Herstellung eines Implantats/eines beschichteten Implantats umfasst zunächst den Schritt des Aufbringens einer Maske auf den Implantatgrundkörper. Die Maske weist derartige Abmaße und Freimachungen auf, dass der mindestens eine berechnete und definierte Oberflächenabschnitt frei liegt und die nicht zu beschichtenden Oberflächenabschnitte mit der Maske bedeckt sind. Vorzugweise ist die mit Freimachungen versehene Maske aus Silikon gefertigt.

Es folgt eine Beaufschlagung des mindestens einen zu beschichtenden Oberflächenabschnitts mit einem Strahlgut. Das bedeutet, dass die freiliegende und nicht bedeckte Oberflache des Implantatgrundkörpers mit dem Strahlgut beaufschlagt, d.h. gestrahlt wird.

Bei dem Strahlgut handelt es sich vorzugsweise um Edelkorund, welches bei Einwirken auf den Grundkörper aus PEEK eine Aufrauung des Oberflächenabschnitts hervorruft. Dieser Vorgang wird bei einem Druck von 1 bis 3 bar, vorzugsweise bei 2 bar durchgeführt.

Die Aufrauung sorgt für eine verbesserte Haftung der in einem anschließenden Schritt aufgetragenen Haftvermittlerschicht auf dem mindestens einen Oberflächenabschnitt des Implantatgrundkörpers. Die Auftragung der Haftvermittlerschicht erfolgt mittels eines vakuumbasierten Beschichtungsverfahrens, d. h. mit einem PVD-Verfahren. Dabei wird eine Schicht mit einer Dicke von 2 - 6 µm, insbesondere einer Dicke von 3 - 5 µm, dichten Titanmaterials auf dem/den Oberflächenabschnitt(en) aufgetragen.

Diese Haftvermittler- oder Zwischenschicht wird in einem weiteren Verfahrensschritt mit einer abschließenden aus reinem Titan bestehenden osteointegrativen Schicht versehen. Aufgetragen wird eine Schicht mit einer Dicke von 50 - 70 µm, insbesondere von 55 - 65 pm, welche eine Porosität von 70 - 90 % und einer Rauheit R_{z} von mindestens 45 µm besitzt.

Das Porous Coating kann beispielsweise mittels eines Elektronenschmelzverfahrens auf die Haftvermittlerschicht aufgebracht werden. In diesem Fall wird schichtweise Sinterpulver, bzw. Titanpulver auf die Haftvermittlerschicht aufgetragen und mittels Energiebeaufschlagung durch eine Strahlenquelle gemäß der jeweiligen Abmaße der Querschnittschicht verschmolzen und im Nachgang gekühlt. Die von der Strahlenquelle ausgegebene Energie trifft dabei nur auf die Pulverteilchen, welche verfestigt werden sollen, also ein Materialteilchen des späteren Implantats darstellen. Daran anschließend wird die nächste Querschnittschicht auf das bereits verschmolzene Material aufgetragen und wiederum mittels Energiebeaufschlagung geschmolzen. Die Bearbeitung erfolgt Schicht für Schicht in vertikaler Richtung.

Das Elektronenschmelzverfahren eignet sich besonders, um die gewünschte Porosität von 70 - 90 % der osteointegrativen Schicht zu erzielen.

Bei durchgeführten Testungen von PEEK-Grundkörpern mit einer Titan-Doppelbeschichtung aus einer Haftvermittlerschicht und einer osteointegrativen Schicht konnten folgende hervorragenden Ergebnisse erzielt werden:

**Tabelle 1: Test Zugfestigkeit nach ASTM F1147**

| | Test Zugfestigkeit nach ASTM F1147 |
|---|---|
| Testlauf 1 (6 Proben) | X = 59,7 MPa σ=4,8 MPa |
| Testlauf 2 (5 Proben) | X = 56,4 MPa o =4,8 MPa |
| Testlauf 3 (5 Proben) | X = 35,6 MPa σ =6,7 MPa |
| Testlauf 4 (5 Proben) | X = 41,5 MPa σ =5,3 MPa |

**Tabelle 2: Test Scherkraft nach ASTM F1044**

| | Test Scherkraft nach ASTM F1044 |
|---|---|
| Testlauf 1 (5 Proben) | X = 37,8 MPa σ = 1,7 MPa |
| Testlauf 2 (5 Proben) | X = 38,2 MPa σ =4,3 MPa |
| Testlauf 3 (5 Proben) | X = 29,I MPa σ =2,8 MPa |

Die Erfindung wird im Folgenden anhand eines Ausführungsbeispiels unter Bezugnahme auf die beigefügten schematischen Zeichnungen naher erläutert.

Hierbei zeigen:
- Fig. 1: eine Schnittdarstellung durch ein erfindungsgemäßes Implantat und
- Fig. 2: eine Draufsicht auf ein Wirbelkörperimplantat.

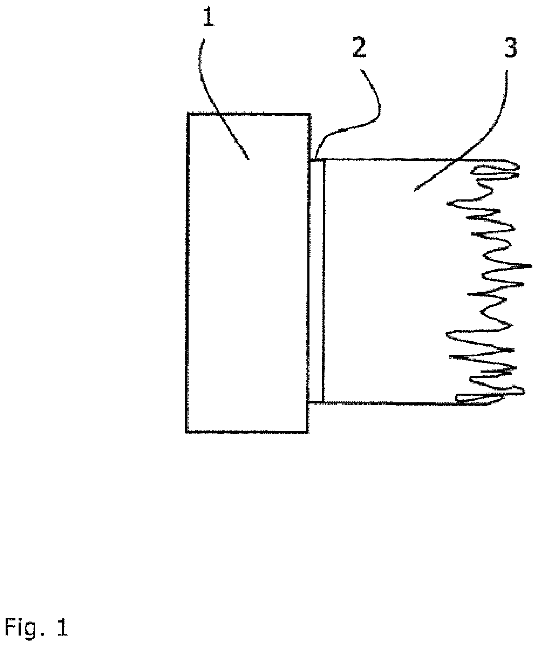

Wie in Fig. 1 dargestellt, ist auf einen Grundkörper 1 des Implantates zunächst eine Haftvermittlerschicht 2 aufgebracht. Diese Haftvermittlerschicht 2 befindet sich auf mindestens einem Oberflächenabschnitt des Grundkörpers 1, wobei die Anzahl sowie die Größe der Oberflächenabschnitte u. a. von den Ausmaßen des Implantats und der Größe, dem Gewicht und dem Geschlecht des Patienten abhängig sind.

Vor dem Aufbringen der Haftvermittlerschicht 2 auf den Oberflächenabschnitt/die Oberflächenabschnitte wird der Grundkörper 1 mit einer Maske abgedeckt. Diese Maske besteht beispielsweise aus Silikon und definiert den zu beschichtenden Oberflächenabschnitt durch Freimachungen, d. h. der Oberflächenabschnitt ist nicht von dem Silikonmaterial der Maske bedeckt.

Der Oberflächenabschnitt wird anschließend mit einem Strahlgut, vorzugweise Edelkorund bei einem Druck von 2 bar beaufschlagt, um eine Aufrauung des Oberflächenabschnittes zu bewirken.

Es folgt die Beschichtung des aufgerauten Oberflächenabschnitts mit einer sehr dichten Haftvermittlerschicht 2 aus reinem Titan. Diese Schicht 2 weist eine Dicke von 3 µm auf, wobei der Vorgang des Auftragens mittels eines PVD-Verfahrens erfolgt.

Der mit einer Haftvermittlerschicht 2 versehene Oberflächenabschnitt wird in einem abschließenden Verfahrensschritt mit einer osteointegrativen Schicht 3 versehen. Diese Schicht weist eine Dicke von im Wesentlichen 60 µm bei einer Porosität von 80 % und einer Rauheit R_{z} von 50 µm auf. Die Aufbringung der osteointegrativen Schicht 3 erfolgt mittels eines Elektronenstrahlschmelzverfahrens auf der Grundlage von Titanpulver.

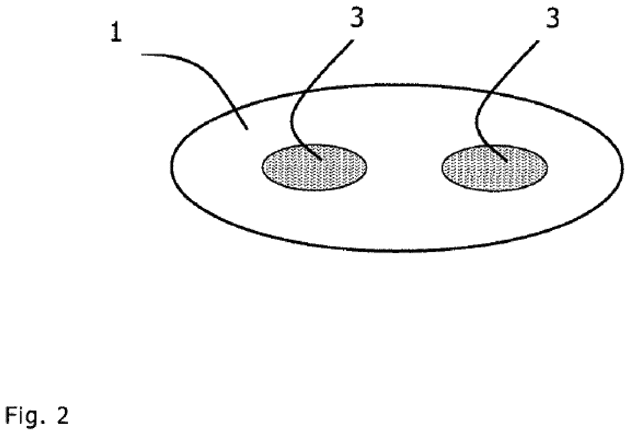

In der Fig. 2 wird schematisch ein Wirbelköperimplantat mit einem Grundkörper 1 dargestellt. Der Draufsicht ist zu entnehmen, dass zwei Oberflächenabschnitte des Grundkörpers eine Beschichtung aufweisen. Die zum Knochen des menschlichen oder tierischen Patienten weisende osteointegrative Schicht 3 besteht folglich aus zwei voneinander beabstandeten Einzelbereichen. Die beim Verfahren zum Aufbringen der Doppelbeschichtung auf das Implantat verwendete Silikonmaske weist zwei Freimachungen in Form der beiden Einzelbereiche der osteointegrativen Schicht 3 auf. Es ist zu beachten, dass bei der Definierung mehrerer zur beschichtenden Oberflächenabschnitte des Grundkörpers die Schichtdicken der jeweiligen Haftvermittlerschichten 2 und osteointegrativen Schichten 3 übereinstimmen, d. h. im Wesentlichen die gleichen Dickenwerte aufweisen. Abweichungen von ± 0,5 µm spielen hingegen keine größere Rolle.

Hinsichtlich eines verbesserten Herstellungsverfahrens stellt die Erfindung gemäß einer ersten weiteren Ausführungsform bereit ein Verfahren zur Herstellung eines Implantats umfassend die Schritte: Aufbringen einer Maske auf einen Grundkörper (1) des Implantats zur Definition von mindestens einem zu beschichtenden Oberflächenabschnitt des Grundkörpers (1), Beaufschlagen des mindestens einen zu beschichtenden Oberflächenabschnitts mit einem Strahlgut, Aufbringen einer aus Titan bestehenden Haftvermittlerschicht (2) mit einer Dicke von 2 - 6 µm, insbesondere von 3 - 5 µm, mittels eines vakuumbasierten Beschichtungsverfahren auf den mindestens einen definierten Oberflächenabschnitt, Aufbringen einer aus Titan bestehenden osteointegrativen Schicht (3) auf die aufgebrachte Haftvermittlerschicht (2) mit einer Dicke von 50 - 70 µm, insbesondere von 55 - 65 µm, einer Porosität von 70 - 90 % und einer Rauheit R_{z} von mindestens 45 µm.

Gemäß einer zweiten weiteren Ausführungsform stellt die Erfindung bereit das Verfahren gemäß der ersten weiteren Ausführungsform, wobei die Beaufschlagung des mindestens einen zu beschichtenden Oberflächenabschnitts mit Strahlgut, mit Edelkorund durchgeführt wird.

Gemäß einer dritten weiteren Ausführungsform stellt die Erfindung bereit das Verfahren gemäß der ersten oder der zweiten weiteren Ausführungsform, wobei die Beaufschlagung des mindestens einen zu beschichtenden Oberflächenabschnitts mit Strahlgut mit einem Druck von 1 - 3 bar, insbesondere mit einem Druck von 2 bar durchgeführt wird.

Gemäß einer vierten weiteren Ausführungsform stellt die Erfindung bereit das Verfahren nach gemäß einer der ersten bis dritten weiteren Ausführungsformen, wobei eine Maske aus Silikon auf den Grundkörper aufgebracht wird.

Gemäß einer fünften weiteren Ausführungsform stellt die Erfindung bereit das Verfahren gemäß einer der ersten bis vierten weiteren Ausführungsformen, wobei die osteointegrative Schicht (3) auf die Haftvermittlerschicht (2) mittels eines Elektronenstrahlschmelzverfahrens aufgebracht wird.

### Bezugszeichenliste

- 1: Grundkörper des Implantats
- 2: Haftvermittlerschicht
- 3: osteointegrative Schicht

## Patentansprüche

1. Implantat mit einem Grundkörper (1) und einer auf mindestens einem Oberflächenabschnitt des Grundkörpers (1) aufgebrachten Doppelbeschichtung, wobei die Doppelbeschichtung aus einer direkt auf dem mindestens einen Oberflächenabschnitt des Grundkörpers aufgebrachten Haftvermittlerschicht (2) und einer, die Haftvermittlerschicht (2) bedeckende osteointegrativen Schicht (3) besteht und diese Schichten (2; 3) aus Reintitan bestehen, die Haftvermittlerschicht (2) eine Dicke von 2 - 6 µm, insbesondere eine Dicke von 3 - 5 µm, die osteointegrative Schicht (3) eine Dicke von 50 - 70 µm, insbesondere eine Dicke von 55 - 65 µm, aufweist, die Haftvermittlerschicht (2) eine hohe Dichte hat und die osteointegrative Schicht (3) eine Porosität von 70 - 90 % und eine Rauheit R_{z} von mindestens 45 µm besitzt.

2. Implantat nach Anspruch 1, **dadurch gekennzeichnet, dass** der Grundkörper (1) aus Polyetheretherketon (PEEK) besteht.

3. Implantat nach Anspruch 1 oder 2, **dadurch gekennzeichnet, dass** die Haftvermittlerschicht (2) eine Dicke von im Wesentlichen 3 µm aufweist.

4. Implantat nach einem der vorangegangenen Ansprüche, **dadurch gekennzeichnet, dass** die Porosität der osteointegrativen Schicht (3) im Wesentlichen 80 % beträgt.

## Claims

1. Implant having a base body (1) and a double coating applied to at least one surface portion of the base body (1), the double coating consisting of an adhesion-promoting layer (2) applied directly to the at least one surface portion of the base body, and an osteointegrative layer (3) covering the adhesion-promoting layer (2), and these layers (2, 3) consisting of pure titanium, the adhesion-promoting layer (2) having a thickness of 2-6 µm, in particular a thickness of 3-5 µm, the osteointegrative layer (3) having a thickness of 50-70 µm, in particular a thickness of 55-65 µm, the adhesion-promoting layer (2) having a high density and the osteointegrative layer (3) having a porosity of 70-90% and a roughness R_{z} of at least 45 µm.

2. Implant according to claim 1, **characterized in that** the base body (1) consists of polyetheretherketone (PEEK).

3. Implant according to claim 1 or 2, **characterized in that** the adhesion-promoting layer (2) has a thickness of substantially 3 µm.

4. Implant according to any one of the preceding claims, **characterized in that** the porosity of the osteointegrative layer (3) is substantially 80%.

## Revendications

1. Un implant présentant un corps de base (1) et un revêtement double appliqué sur au moins une partie de surface du corps de base (1), dans lequel le revêtement double consiste en une couche de promoteur d'adhérence (2) appliquée directement sur l'au moins une partie de surface du corps de base et en une couche ostéointégrative (3) recouvrant la couche de promoteur d'adhérence (2), et ces couches (2, 3) consistent en titane pur, la couche de promoteur d'adhérence (2) présente une épaisseur de 2-6 µm, en particulier une épaisseur de 3-5 µm, la couche ostéointégrative (3) présente une épaisseur de 50-70 µm, en particulier une épaisseur de 55-65 µm, la couche de promoteur d'adhérence (2) présente une densité élevée et la couche ostéointégrative (3) présente une porosité de 70-90 % et une rugosité R_{z} d'au moins 45 µm.

2. L'implant selon la revendication 1, **caractérisé en ce que** le corps de base (1) consiste en polyétheréthercétone (PEEK).

3. L'implant selon la revendication 1 ou 2, **caractérisé en ce que** la couche de promoteur d'adhérence (2) présente une épaisseur d'essentiellement 3 µm.

4. L'implant selon l'une quelconque des revendications précédentes, **caractérisé en ce que** la porosité de la couche ostéointégrative (3) est essentiellement de 80%.
